# EUROPEAN PATENT APPLICATION

(11) **EP 1 867 353 A1**
(43) Date of publication of application: **19.12.2007**
(21) Application number: 06730779.3
(22) Date of filing: 31.03.2006
(51) Int. Cl.: A61M 1/14, B01D 61/30, B01D 65/02

(54) **HEMODIALYZER**

(30) Priority: 01.04.2005 JP 2005106646
(71) Applicant: JMS Co., Hiroshima-shi, Hiroshima 730-0812 (JP); Kitakyushu Institute of Biophysics Co.,Ltd., Kitakyushu-shi, Fukuoka 807-0831 (JP)
(72) Inventor: KIM, Sung-Teh, Kitakyushu-shi, Fukuoka 8070831 (JP); YAMAMOTO, Chieko, Kitakyushu-shi, Fukuoka 8070833 (JP); SEGAWA, Kayoko, Espoir Asakawa 205, Kitakyushu-shi, Fukuoka 8070879 (JP); YAMANAKA, Kunihiko, c/o JMS CO., Hiroshima-shi, Hiroshima 7300812 (JP); MASAOKA, Katsunori, c/o JMS CO., Hiroshima-shi, Hiroshima 7300812 (JP)
(74) Representative: Vossius & Partner
(86) International application number: PCT/JP2006/306831
(87) International publication number: WO 2006/106916

(57) **Abstract**

Provided is a hemodialyzer in which deterioration in membrane performance of a hollow fiber with time can be grasped accurately during dialysis and the performance can be recovered quickly and positively. The hemodialyzer, constituted by connecting an artery side blood circuit (2) having a blood pump (7) to the artery side of a dialyzer (1), connecting a vein side blood circuit (3) to the vein side of the dialyzer (1), and connecting a dialysate supply line (4) and a dialysate discharge line (5), respectively, to the side face of the dialyzer (1), is provided with a detector (15) for determining the pressure in the blood circuit, and a detector (16) for determining the pressure in the dialysate line, in which transmembrane pressure is calculated based on the determined pressures, and reverse filtering is performed by the dialyzer (1) when the transmembrane pressure exceeds a predetermined level. Reverse filtering is performed by flushing operation and clogging of pores of the dialyzing membrane in the dialyzer (1) is eliminated thus recovering performance of the dialyzer (1).

## Description

### TECHNICAL FIELD

The present invention relates to a hemodialyzer which purifies the blood utilizing extracorporeal circulation as a treatment which substitutes for the function of the kidney of a chronic renal failure patient in the medicine field.

### BACKGROUND ART

For a treatment of a patient suffering from renal failure, there have been proposed various blood purifying methods in which blood is taken out from the living body of the patient to be purified and the purified blood is then returned into the body. For example, the blood purification methods utilizing extracorporeal circulation are classified into the following types:
hemodialysis (HD) by diffusion, hemofiltration (HF) which performs body fluid removal/substitution by ultrafiltration, and hemodialysis filtration (HDF) in which HD and HF are combined.

In any one of the above-mentioned methods, the hemodialyzer including cylindrical housing filled with a plurality of semipermeable membranes (referred to as "dialysis membrane") such as a hollow fiber cellulose membrane, polyacrylonitrile membrane, or polysulfone membrane, which purifies the blood by diffusion and/or filtration between the blood and a dialysate through the dialysis membrane, is mainly employed as an essential method.

Detection (monitoring) of a clogging of a pore of the hollow fiber membrane which directly purifies the blood is important so that a dialyzer may always be used in a suitable condition. When a dialysis treatment is continued, the clogging of a pore of a membrane is presumably caused by adhesion (fouling) of proteins produced temporally, and deposition of a thrombus, etc., which remarkably deteriorates an inherent function of the dialyzer.

In recent years, a hemodialyzer with enhanced internal filtration which removes solute by aggressively utilizing an internal filtration phenomenon caused by the pressure gradient in a dialyzer attracts attention as an efficient blood purification method. The internal filtration enhanced dialyzer is constructed of the same constitutional elements as that of a common hemodialysis (HD), and the treatment manner is apparently hardly different from that of HD. However, for internal filtration enhancement, it is necessary to narrow an inner diameter of a hollow fiber, lengthen an effective length of a hollow fiber, increase a filling ratio of a hollow fiber membrane, narrow an inner diameter of a housing, etc. Because of such a structure, in particular, the above-mentioned clogging of a membrane pore is liable to occur, and therefore, development of a measure for immediately and positively eliminating the clogging is urged.

Conventionally, various measures for preventing deterioration with time of a dialyzer have been proposed. For example, a method in which a dialyzer whose performance is deteriorated due to corpuscle components or proteins adhering to the membrane surface after use is washed with a pharmaceutical drug; a measure disclosed in Patent document 1 in which removal of proteins adsorbed and deposited is facilitated by employing a selective separation membrane in which a water permeation rate in physiological saline is 1.07 times or more as a water permeation rate in pure water; and a method disclosed in Patent document 2 in which, in a hollow fiber membrane equipped with a blood inflow part/outflow part and a dialysate inflow part/outflow part, the pressure of at least one of the blood inflow part/outflow part and the dialysate inflow part/outflow part is determined, and then the change with time of the determined pressure is analyzed, thereby detecting a clogging state of the filer of the hollow fiber membrane.
Patent Document 1: JP 2003-38940 A
Patent Document 2: JP 2004-248844 A

### DISCLOSURE OF THE INVENTION

### PROBLEMS TO BE SOLVED BY THE INVENTION

However, as to recovery of the deteriorated performance, the method in which the dialyzer is washed with the above-mentioned pharmaceutical drug is insufficient. The separation membrane of Patent Document 1 provides an improvement of a membrane itself, and deterioration of membrane performance and recovery from the deterioration during dialysis are not described in Patent Document 1. Thus, it cannot always be thought that the separation membrane of Patent Document is effective. Further, Patent Document 2 discloses that a clogging of a hollow fiber membrane is detected, and a dosage amount of anti-coagulant is properly controlled, thereby preventing the filter clogging from accelerating, but does not disclose recovery of the deteriorated membrane performance.

The present invention aims to solve the problems of the related art examples, and provides a hemodialyzer which can correctly monitor deterioration with time in the performance of a hollow fiber membrane during dialysis, and can immediately and positively recover the deteriorated performance. In particular, the present invention provides a hemodialyzer which can enhance and maintain internal filtration in a favorable condition by applying the hemodialyzer of the present invention to the internal filtration enhanced hemodialyzer.

### MEANS FOR SOLVING THE PROBLEMS

To solve the above-mentioned problems, a hemodialyzer according to the present invention is characterized by including: an artery side blood circuit having a blood pump that is connected to an artery side of a dialyzer; a vein side blood circuit that is connected to a vein side of the dialyzer; a dialysate supply line and a dialysate discharge line that are connected to one side of the dialyzer, respectively; a detector for determining a pressure in the blood circuit; and a detector for determining a pressure in the dialysate line, and characterized in that a transmembrane pressure is calculated based on pressures determined-detection with these detectors, and when the transmembrane pressure increases by a given value or more, a clogging of a pore of a dialysis membrane is eliminated by reverse filtration in the dialyzer, and a performance of the dialyzer is recovered.

Measurement of the pressure in the blood circuit by the detector is performed by determining the pressure in a vein side blood circuit and/or an artery side blood circuit. In measurement of the pressure in the vein side blood circuit and the artery side blood circuit, the average pressure of both the circuits is used for calculating the trans-membrane pressure difference. Measurement of the pressure in a dialysate line by the detector is performed by determining the pressure in a dialysate supply line and/or a dialysate discharge line. In measurement of the pressure in the dialysate supply line and the dialysate discharge line, the average pressure of both the lines is used for calculating the trans-membrane pressure difference.

In addition, the hemodialyzer of the present invention further includes: a first liquid supply unit and a second liquid supply unit provided to the dialysate supply line and the dialysate discharge line, respectively; a bypass line which communicates an upstream and a downstream of the liquid supply unit of the line provided to at least one line of the dialysate supply line and the dialysate discharge line; a third liquid supply unit which can adjust a flow rate of dialysate for water elimination/water supplementation and can supply liquid in both forward and reverse directions provided to the bypass line, and is characterized in that reverse filtration is performed using the third supply liquid unit.

Further, in the present invention, when the trans-membrane pressure difference, which is calculated from inputting measurement values obtained from the detector determining the pressure in the blood circuits and the detector determining the pressure in the dialysate lines, becomes equal to or more than a given value, it is preferable to provide a controller which outputs an instruction of reverse filtration. It is also preferable that the instruction of reverse filtration output from the controller be output to a third liquid supply unit. In addition, in the dialyzer according to the present invention, it is especially effective that the dialyzer is a dialyzer with enhanced internal filtration.

### EFFECT OF THE INVENTION

According to the hemodialyzer of the present invention, by detecting the pressure difference between the pressure at the blood side and the pressure at the dialysate side, even during dialysis, the clogging of the pore of the dialysis membrane of the dialyzer can be detected, and impurities which cause the clogging, such as a protein or thrombus, can be removed from the membrane pore by immediately performing reverse filtration, which positively contributes to the recovery of the function of the dialyzer.

### BRIEF DESCRIPTION OF THE DRAWINGS

Fig. 1 is a schematic diagram illustrating an example of an outline of a hemodialyzer suitable for applying the present invention.
Fig. 2 is a diagram illustrating the timing of flushing in carrying out the present invention.
Fig. 3 is a diagram specifically illustrating an actual flow and flow amount of a blood and a dialysate according to Examples of the present invention.

### DESCRIPTION OF REFERENCE NUMERALS

1 dialyzer, 2 artery side blood circuit,
3 vein side blood circuit, 4 dialysate supply line,
5 dialysate discharge line,
6a artery side connection part,
6b vein side connection part, 7 BLOOD PUMP,
8 artery side chamber, 9 overflow line,
10 vein side chamber, 11 first liquid supply unit,
12 second liquid supply unit, 13 bypass line,
14 third liquid supply unit, 15 venous pressure detector,
16 arterial pressure detector, 17 controller

### BEST MODE FOR CARRYING OUT THE INVENTION

Hereinafter, the present invention will be described in detail.

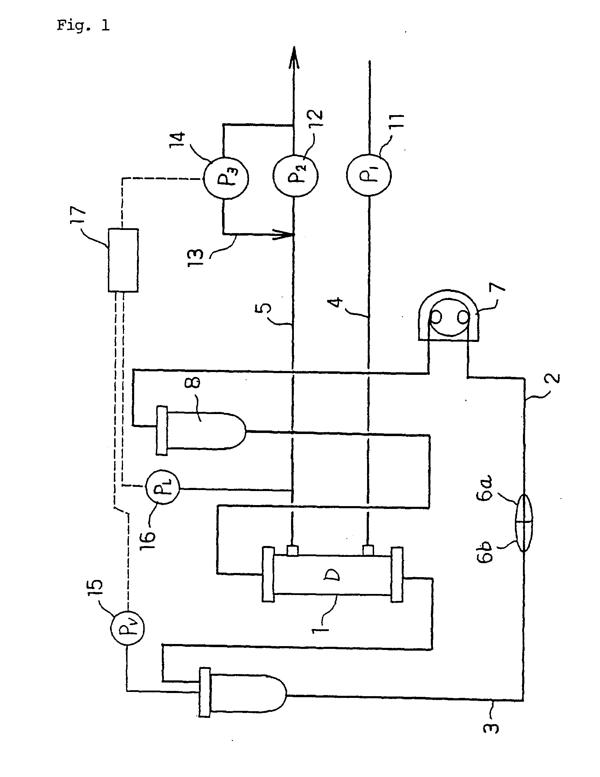

Fig. 1 is a diagram illustrating an example of a hemodialyzer suitable for applying the present invention, in which: an artery side blood circuit 2 is connected to an artery side of a dialyzer 1 which performs blood purification by extracorporeal circulation of blood; a vein side blood circuit 3 is connected to the vein side of the dialyzer 1; and a dialysate supply line 4 and a dialysate discharge line 5 are connected to either one side of the dialyzer 1, respectively. The artery side blood circuit 2 has a blood pump 7 for collecting the blood from the artery side connection part 6a of a patient, and introducing the collected blood to the dialyzer 1, and an artery side chamber 8 which has functions of removing bubbles mixed in the circuit, etc. A vein side blood circuit 3 for returning the purified blood to the vein side connection part 6b of a patient has a vein side chamber 10.

The dialysate supply line 4 and the dialysate discharge line 5 are provided with a first liquid supply unit 11 (dialysate supply side) and a second liquid supply unit 12 (dialysate discharge side), respectively. Used as the first liquid supply unit 11 and the second liquid supply unit 12 are a diaphram pump, a dual pump, etc., which can repeatedly supply liquid in a given amount at a constant rate with high degree of accuracy. The first liquid supply unit 11 and the second liquid supply unit 12 are operated while, for example, being interlocked with each other in such a manner that the amount of supply liquid of each unit is always the same. In the case of eliminating water, the first liquid supply unit 11 and the second liquid supply unit 12 may be operated in such a manner that the amount of liquid supply of the second liquid supply unit 12 is larger than that of supply of the first liquid supply unit 11.

A bypass line 13 which communicates the upstream side and the downstream side of the second liquid supply unit 12 in the dialysate discharge line 5 is further provided. The bypass line 13 is provided with a third liquid supply unit 14 which can adjust the flow amount of the dialysate for water elimination/water supplementation and can supply liquid in both forward and reverse directions. An example shown in the figure describes a case where the bypass line which can supply liquid in both forward and reverse directions is provided only on the dialysate discharge line 5. However, the present invention is not limited thereto, and the bypass line which can supply liquid in both forward and reverse directions can be provided only on the dialysate supply line 4, or on both the dialysate supply line 4 and the dialysate discharge line 5.

In a case where hemodialysis is practically carried out using the dialyzer structured as described above, hemodialysis is performed through the following steps: a priming process which cleans the blood circuit or the dialysate circuit before starting a treatment; a blood collection process after puncture for collecting the blood from the inside of the body; a dialysis process by diffusion and filtration in the dialyzer for performing extracorporeal circulation; and a blood returning process for returning the blood in the blood circuits into the inside of the body.

The present invention is useful as a hemodialyzer with enhanced internal filtration. The inventors of the present invention carried out many experimental studies for enhancing and maintaining internal filtration. As a result, the inventors acquired the following fact described below, and found that the mechanical action of a dialyzer can be utilized. More specifically, as one of the causes of decrease with time in an internal filtration flow rate, lowering of the ultrafiltration rate (UFR) due to fouling of a membrane pore, and the like is presumably involved. In usual, the amount of ultrafiltration is determined by a transmembrane pressure (TMP: pressure obtained by subtracting the pressure at the dialysate side from the pressure at the blood side). In view of this, in the present invention: a detector 15 which detects the pressure (P_{V}) in a vein side chamber 10 and a detector 16 which detects the pressure (P_{L}) in the dialysate discharge line 5 are provided as shown in Fig. 1; TMP is always monitored for determining UFR; and when the UFR is lowered, a reverse filtration mechanism of the dialyzer is utilized for flushing a pore of a membrane, thereby maintaining the internal filtration flow rate.

For example, the TMP is obtained by inputting the pressure from the detectors 15 and 16, and the UFR is calculated based on TMP. When the UFR remarkably decreases (i.e., the TMP remarkably increases), a clogging of the pore of the hollow fiber of the dialyzer occurs, and it is judged that the performance of the dialyzer is deteriorated. Thus, a measure for eliminating the clogging by a mechanical action is taken. To be specific, when the UFR is lower than a given value (set value), flushing of the membrane pore of the hollow fiber is performed by using the reverse filtration mechanism of the dialyzer, thereby eliminating the clogging for recovering the performance of the dialyzer and maintaining the internal filtration flow rate. In Fig. 1, signals from the detectors 15 and 16 are input to a controller 17, and |P_{V}-P_{L}| is calculated by the controller 17. When the calculated value exceeds a predetermined value, an instruction of reversing rotation is given to the third liquid supply unit 14 to perform reverse filtration, thereby eliminating the clogging. It is also possible to carry out reverse filtration based on a calculated UFR.

The TMP at the time of starting a hemodialysis treatment is determined by parameters such as a type of dialyzer, blood properties of a patient, water elimination rate. Further, after hemodialysis filtration (HDF), a substitution liquid rate in a case of a dilution method becomes an important factor parameter. Due to the above-mentioned conditions, the TMP after dialysis was commenced increases due to the change in the blood properties, clogging of the membrane of the dialyzer, etc., with the progress of the dialysis. In this case, when the TMP increases by an arbitrarily determined pressure within the range of 30 to 300 mmHg, the flushing operation of the dialyzer by the reverse filtration is initiated. The flushing amount and flushing rate in this case varies depending on the dimension (membrane area) and blood flow amount, etc. of the dialyzer, and it is possible to appropriately adjust the flushing amount within the range of 30 to 200 mL and the flushing rate within the range of 50 to 300 mL/min.

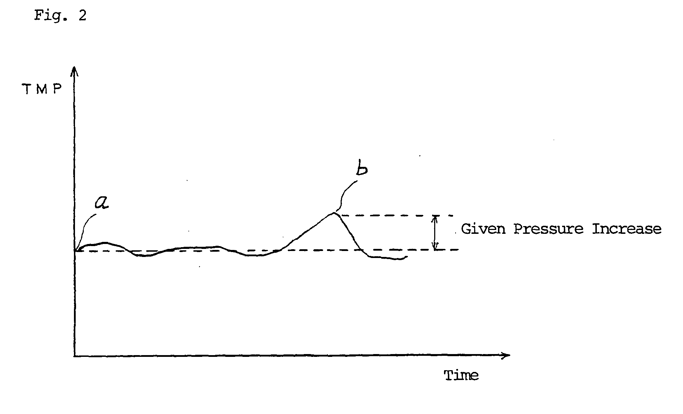

In Fig. 2, the TMP changes with the progress of the dialysis treatment from the time of starting the dialysis treatment (point a). Flushing by reverse filtration is performed when the TMP increases, for example, by 30 mmHg or more (point b), resulting in lowering the TMP. When the TMP reaches the point b and the flushing is initiated, flushing can be started immediately when the TMP reaches the point b, or flushing can be started when the increase in the TMP by 30 mmHg or more at the point b continues over a given period of time.

### Example 1

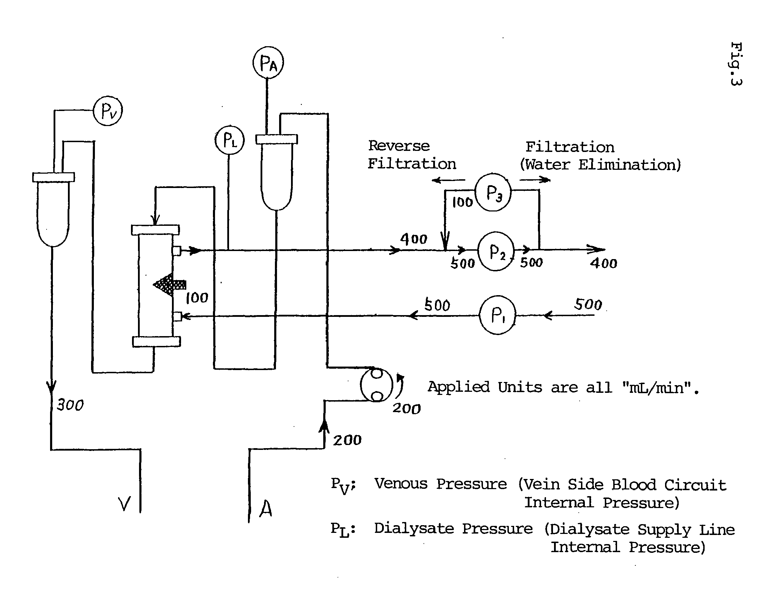

Fig. 3 illustrates an actual flow direction and flow amount (mL/min) of the blood and a dialysate in the blood circuit and the dialysate discharge line shown in Fig. 1. The TMP (UFR may suffice) after dialysis initiation is memorized, and when the TMP, which increases with the progress of dialysis treatment, reaches a given value (30 to 300 mmHg), a flushing operation is commenced.

During the dialysis treatment, water is eliminated by operating the third liquid supply unit rightward (liquid discharge direction, which is opposite to the arrow shown in Fig. 1) at a given water elimination rate. The TMP is calculated as a difference between the venous pressure (pressure in the vein side blood circuit) and the dialysate pressure (pressure in the dialysate supply line).
Alternatively, when the measurement of circuit internal pressure is equipped with the venous pressure P_{V} and the arterial pressure P_{A}, the TMP is calculated by defining the blood circuit internal pressure as a difference between the dialysate pressure and the average of P_{V} and P_{A}.

When the TMP increases by a given pressure relative to the value after the dialysis was commenced, or when the increase state continues over a given period of time, the third liquid supply unit is operated in the direction of reverse filtration (direction of the arrow of Fig. 1) at a predetermined flushing rate. When the amount of liquid supply reaches a given flushing amount, the flushing operation is stopped. After this operation is complete, a usual dialysis treatment condition is returned. After this operation, when the TMP rises again, the above-mentioned flushing operation is repeated.

## Claims

1. A hemodialyzer, comprising:
an artery side blood circuit having a blood pump that is connected to an artery side of a dialyzer;
a vein side blood circuit that is connected to a vein side of the dialyzer;
a dialysate supply line and a dialysate discharge line that are connected to one side of the dialyzer, respectively;
a detector for determining a pressure in the blood circuit; and
a detector for determining a pressure in the dialysate line,
wherein a transmembrane pressure is calculated based on pressures determined-detection with these detectors, and when the transmembrane pressure increases by a given value or more, a clogging of a pore of a dialysis membrane is eliminated by reverse filtration in the dialyzer, and a performance of the dialyzer is recovered.

2. The hemodialyzer according to claim 1, further comprising:
a first liquid supply unit and a second liquid supply unit provided to the dialysate supply line and the dialysate discharge line, respectively;
a bypass line which communicates an upstream side and a downstream of the liquid supply unit of the line provided to at least one line of the dialysate supply line and the dialysate discharge line;
a third liquid supply unit which can adjust a flow rate of dialysate for water elimination/water supplementation and can supply liquid in both forward and reverse directions provided to the bypass line, wherein reverse filtration is performed using the third supply liquid unit.

3. The hemodialyzer according to claim 1 or 2, further comprising a controller for outputting an instruction of reverse filtration when a transmembrane pressure becomes equal to or more than a predetermined value, the transmembrane pressure being calculated from inputting measurement values obtained from the detector for determining the pressure in the blood circuit and the detector for determining the pressure in the dialysate line.

4. The hemodialyzer according to claim 3, wherein an instruction of reverse filtration from the controller is input to the third liquid supply unit.
